(19) **Europäisches Patentamt / European Patent Office / Office européen des brevets**

(11) **EP 4 631 975 A1**

(12) **EUROPEAN PATENT APPLICATION**
published in accordance with Art. 153(4) EPC

(43) Date of publication:
**15.10.2025 Bulletin 2025/42**

(51) International Patent Classification (IPC):
**C07K 16/18** (2006.01) **A61P 25/28** (2006.01)
**A61K 39/00** (2006.01)

(21) Application number: **23901070.5**

(22) Date of filing: **05.12.2023**

(52) Cooperative Patent Classification (CPC):
**A61K 39/00; A61P 25/28; C07K 16/18**

(86) International application number:
**PCT/KR2023/019923**

(87) International publication number:
**WO 2024/123052 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.12.2022 KR 20220171003**

(71) Applicants:
• **Adel Inc.**
  **Seoul 05505 (KR)**

• **Oscotec Inc.**
  **Seongnam-si, Gyeonggi-do 13488 (KR)**

(72) Inventor: **CHO, Mi Hyang**
**Seoul 05505 (KR)**

(74) Representative: **Ullrich & Naumann PartG mbB**
**Schneidmühlstrasse 21**
**69115 Heidelberg (DE)**

Remarks:
The complete document including Reference Table(s) and the Sequence Listing(s) can be downloaded from the EPO website

(54) **FUSION COMPRISING ANTI-TAU ANTIBODY AND PEPTIDE AND USE THEREOF**

(57) The present invention relates to a fusion and use thereof, the fusion comprising an anti-tau antibody or an antigen-binding fragment thereof and a blood-brain barrier receptor-binding peptide. The fusion of the present invention specifically binds to tau proteins by passing through the blood-brain barrier, and thus can effectively prevent or treat neurodegenerative diseases mediated by tau proteins.

[FIG. 7A]

Linker-free fusion (batch# 1, 2, 3)

EP 4 631 975 A1

## Description

### Technical Field

This application claims priority to Korean Patent Application No. 2022-0171003, filed on December 8, 2022, the disclosure of which is incorporated herein by reference in its entirety.

Sequence listing

This application includes a sequence listing that has been submitted electronically in XML format and is incorporated by reference herein in its entirety. A copy of the sequence listing, created on November 28, 2023, is named KC23138-SEQ.xml and is 44.6 kilobytes in size.

The present disclosure relates to a fusion comprising an anti-tau antibody and a peptide, and a use thereof. In particular, the present disclosure relates to a fusion having improved blood-brain barrier permeability and a use thereof for prevention or treatment of a neurodegenerative disease.

### Background Art

Tau protein is a protein that stabilizes the microtubule which is a protein that transports a cellular material. The tau protein exists in six isoforms in the human body and is abundant in neurons of the central nervous system. It is known that in a case where a mutation occurs in the tau protein, the tau protein is hyperphosphorylated, which allows neurofibrillary tangles (NFTs) to be abnormally accumulated in nerve cells, thereby causing a neurodegenerative disease such as dementia, Parkinson's disease, and tauopathy. Accordingly, development of therapeutic agents targeting tau protein has been reported (Korea Patent Application No. 10-2020-0086341).

Meanwhile, brain penetration of neurological disorder drugs, for example, large biotherapeutic drugs such as antibodies or small molecule drugs having low brain penetration, is strictly limited by the extensive and impermeable blood-brain barrier (BBB) together with other cellular components in the neurovascular unit. Previous studies have reported that only a very small percentage (approximately 0.1%) of IgG injected in the bloodstream are able to penetrate into the central nervous system compartment (Felgenhauer, Klin. Wschr. 52 1158-1164 (1974)).

Therefore, there is a need for a tau-targeting drug that can cross the BBB and be effectively delivered to the brain.

### Disclosure of Invention

### Technical Problem

The object of the present disclosure is to solve all of the above-mentioned problems.

Another object of the present disclosure is to provide a fusion comprising an anti-tau antibody or an antigen-binding fragment thereof that specifically binds to tau protein and a blood-brain barrier receptor-binding peptide.

Yet another object of the present disclosure is to provide a polynucleotide encoding the fusion, an expression vector, and a host cell.

Still yet another object of the present disclosure is to provide a method for preparing the fusion.

Still yet another object of the present disclosure is to provide a pharmaceutical composition for preventing or treating a neurodegenerative disease, comprising the fusion.

Still yet another object of the present disclosure is to provide a method for preventing or treating a neurodegenerative disease, comprising administering the fusion to a subject in need of prevention or treatment of a neurodegenerative disease.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided a fusion comprising (i) an anti-tau antibody or an antigen-binding fragment thereof that specifically binds to tau protein and (ii) a blood-brain barrier (BBB) receptor-binding peptide.

In an embodiment, there is provided a fusion in which the anti-tau antibody is directly coupled to the blood-brain barrier receptor-binding peptide without a linker.

In another embodiment, the blood-brain barrier receptor-binding peptide may comprise one or more selected from the group consisting of Angiopep-2 (APEP), RVG29, and TfR binding peptide (TfR).

In yet another embodiment, APEP may comprise, consist of, or consist essentially of the amino acid sequence of SEQ ID NO: 20.

In still yet another embodiment, RVG29 may comprise, consist of, or consist essentially of the amino acid sequence of SEQ ID NO: 22.

In still yet another embodiment, TfR may comprise, consist of, or consist essentially of the amino acid sequence of SEQ ID NO: 24.

In still yet another embodiment, the anti-tau antibody or antigen-binding fragment thereof may comprise a heavy chain variable region (VH) that comprises heavy chain CDR1 comprising the amino acid sequence of SEQ ID NO: 2, heavy chain CDR2 comprising the amino acid sequence of SEQ ID NO: 4, and heavy chain CDR3 comprising the amino acid sequence of SEQ ID NO: 6; and a light chain variable region (VL) that comprises light chain CDR1 comprising the amino acid sequence of SEQ ID NO: 10, light chain CDR2 comprising the amino acid sequence of SEQ ID NO: 12, and light chain CDR3 comprising the amino acid sequence of SEQ ID NO: 14.

In an embodiment, the anti-tau antibody or antigen-binding fragment thereof may bind to an epitope comprising the amino acids at positions 275 to 286 of the wild-type tau protein of SEQ ID NO: 25, in which the amino acid at position 280 is acetylated.

In an embodiment, the anti-tau antibody or antigen-binding fragment thereof may comprise a heavy chain variable region comprising the amino acid sequence of SEQ ID NO: 8 and a light chain variable region comprising the amino acid sequence of SEQ ID NO: 16.

In an embodiment, the anti-tau antibody or antigen-binding fragment thereof may comprise (i) a heavy chain variable region comprising an amino acid sequence selected from the group consisting of SEQ ID NO: 8, SEQ ID NO: 27, SEQ ID NO: 29, SEQ ID NO: 31, and SEQ ID NO: 33; and (ii) a light chain variable region comprising an amino acid sequence selected from the group consisting of SEQ ID NO: 16, SEQ ID NO: 35, SEQ ID NO: 37, and SEQ ID NO: 39.

In another embodiment, there is provided a fusion in which the anti-tau antibody comprises a light chain and a heavy chain, and the blood-brain barrier receptor-binding peptide is linked to Fc region of the heavy chain.

In yet another embodiment, the fusion may be a fusion that crosses the blood-brain barrier and binds specifically to tau protein.

In still yet another embodiment, the anti-tau antibody or antigen-binding fragment thereof may be any one selected from the group consisting of full-length antibody, Fab, scFv, F(ab')2, and Fv.

In still yet another embodiment, the anti-tau antibody may be an IgG antibody.

According to another aspect of the present disclosure, there is provided a polynucleotide encoding the fusion disclosed herein or heavy and/or light chains thereof.

According to yet another aspect of the present disclosure, there is provided an expression vector comprising the polynucleotide.

According to still yet another aspect of the present disclosure, there is provided a host cell that comprises a polynucleotide encoding the fusion disclosed herein or an expression vector comprising the polynucleotide.

According to still yet another aspect of the present disclosure, there is provided a method for producing the fusion disclosed herein, comprising culturing a host cell that comprises a polynucleotide encoding the fusion or light chain and heavy chain polypeptides included in the fusion.

According to still yet another aspect of the present disclosure, there is provided a pharmaceutical composition for preventing or treating a neurodegenerative disease, comprising the fusion disclosed herein.

According to still yet another aspect of the present disclosure, there is provided a method for preventing or treating a neurodegenerative disease, comprising administering an effective amount of the fusion or pharmaceutical composition disclosed herein to a subject in need of prevention or treatment of a neurodegenerative disease.

In an embodiment, the neurodegenerative disease may be selected from the group consisting of tauopathy, primary age-related tauopathy, chronic traumatic encephalopathy, progressive supranuclear palsy, corticobasal degeneration, frontotemporal dementia, Lytico-Bodig disease, Parkinson's disease, subacute sclerosing meningitis, lead encephalopathy, tuberous sclerosis, ganglioglioma, gangliocytoma, meningioangiomatosis, subacute sclerosing panencephalitis, Hallervorden-Spatz disease, and lipofuscinosis.

In another embodiment, the tauopathy may be selected from the group consisting of Alzheimer's disease, progressive supranuclear palsy, corticobasal degeneration, Pick's disease, a group of related disorders collectively termed frontotemporal dementia with parkinsonism linked to chromosome 17 (FTDP-17), amyotrophic lateral sclerosis, Creutzfeldt-Jakob disease, dementia pugilistica, Gerstmann-Straussler-Scheinker syndrome, Lewy body disease, chronic traumatic encephalopathy, and Huntington's disease.

In yet another embodiment, the fusion or pharmaceutical composition disclosed herein may be administered via any one route of administration selected from the group consisting of intramuscular, intravenous, intraarterial, intraperitoneal, transdermal, subcutaneous, intradural, intracerebral, intracerebroventricular, intrapulmonary, or intranasal administration.

**Advantageous Effects of Invention**

As disclosed herein, the fusion prepared by coupling an anti-tau antibody with a blood-brain barrier receptor-binding peptide exhibits an excellent effect in enabling the anti-tau antibody to effectively cross the BBB. In particular, the anti-tau antibody exhibited superior BBB permeability in a case of being directly linked to the blood-brain barrier receptor-binding peptide without a linker. The fusion polypeptide of the present disclosure is capable of inhibiting abnormal aggregation of tau protein or effectively preventing or treating a neurodegenerative disease.

**Brief Description of Drawings**

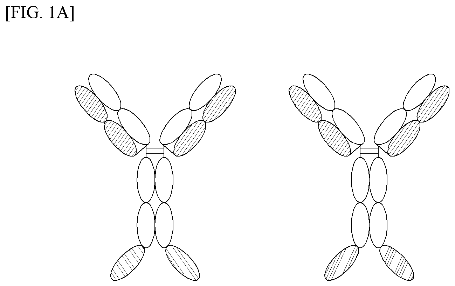

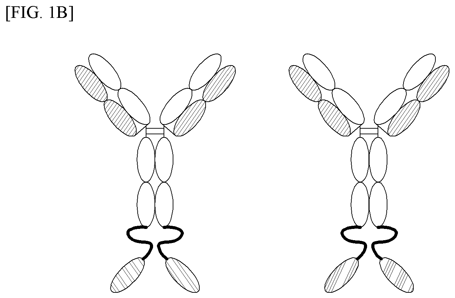

Fig. 1 illustrates schematic diagrams of fusion proteins according to an embodiment of the present disclosure. Fig. 1A illustrates a fusion protein in which an anti-tau antibody is directly linked to a blood-brain barrier receptor-binding peptide without a linker, and Fig. 1B illustrates a fusion protein in which an anti-tau antibody is linked to a blood-brain barrier receptor-binding peptide via a linker.

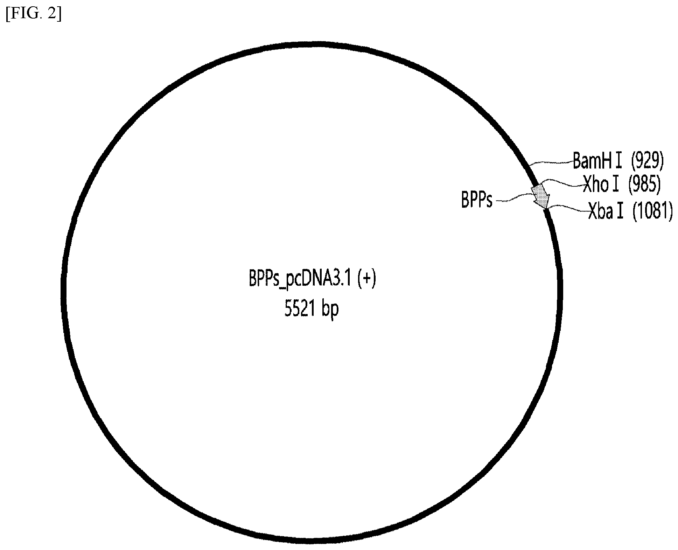

Fig. 2 illustrates pcDNA3.1(+) vector for cloning three blood-brain barrier (BBB) receptor-binding peptides according to an embodiment of the present disclosure. In the present specification, the term "blood-brain barrier receptor-binding peptide" is used interchangeably with "brain penetrating peptide (BPP)" and is denoted as BPP in the drawings.

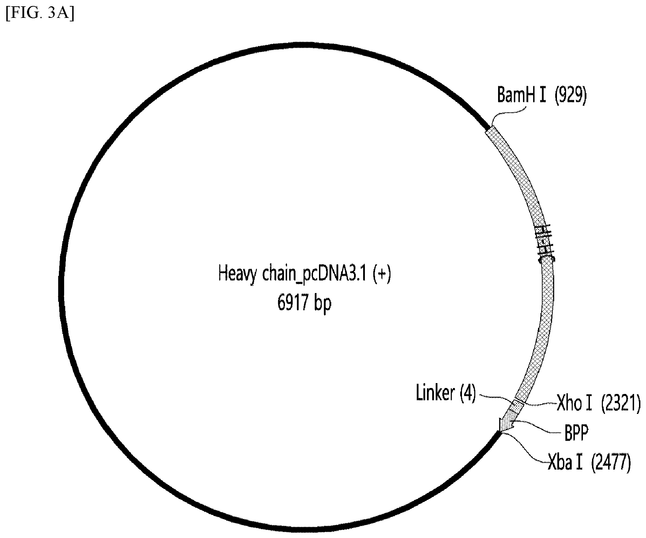

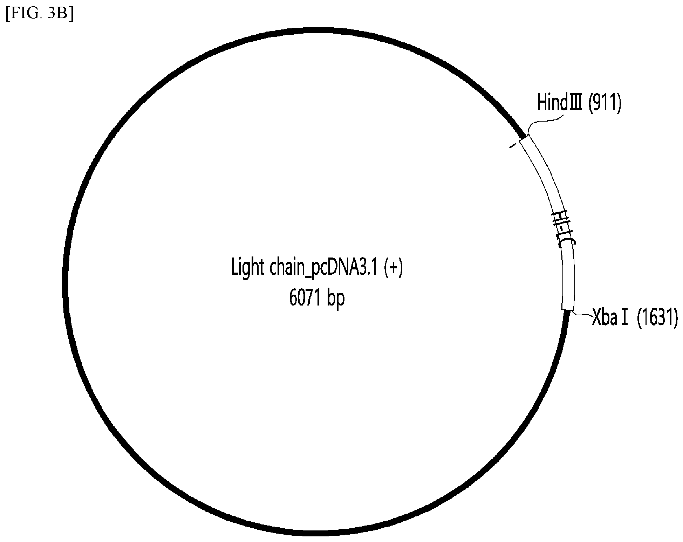

Fig. 3 illustrates diagrams showing expression vectors (pcDNA3.1(+)) into which the heavy and light chain genes of the H1 antibody (a human anti-tau antibody) have been inserted, respectively. Fig. 3A illustrates a diagram showing an expression vector in which the heavy chain gene of the H1 antibody is inserted upstream of a brain penetrating peptide (or a linker-brain penetrating peptide) using the restriction enzymes BamHI and XhoI; and Fig. 3B illustrates a diagram showing an expression vector in which the light chain gene of the H1 antibody is inserted using the restriction enzymes HindIII and XbaI.

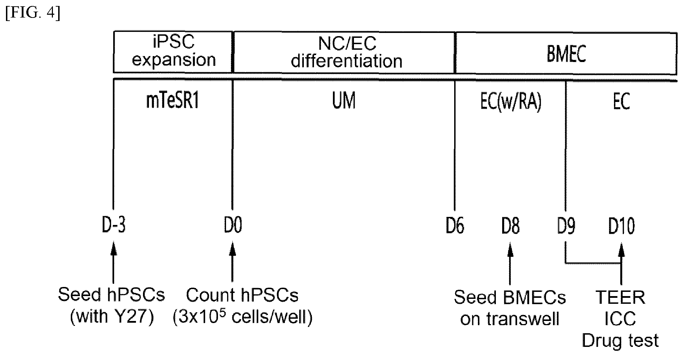

Fig. 4 illustrates a diagram showing a protocol for differentiation of induced pluripotent stem cells ("iPSCs") into human brain microvascular endothelial cells ("BMECs").

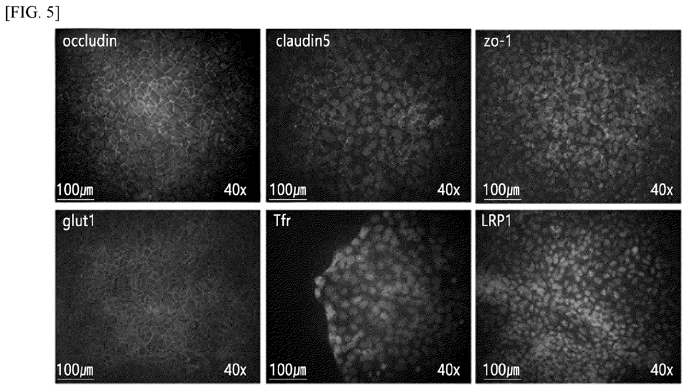

Fig. 5 illustrates images obtained by fluorescence microscopy (magnification: 400x) on days 6 to 10 after induction of differentiation into iBMECs, showing expression of BBB-associated proteins (scale bar: 100 $\mu$m).

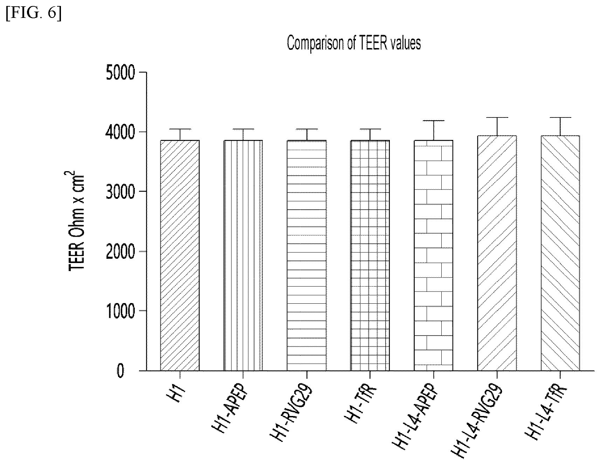

Fig. 6 illustrates a graph showing the results obtained by measuring the TEER values of respective transwells administered with one antibody and six fusions according to embodiments of the present disclosure. L4 indicates a linker, and the values are represented in units of $\Omega$ x cm$^2$.

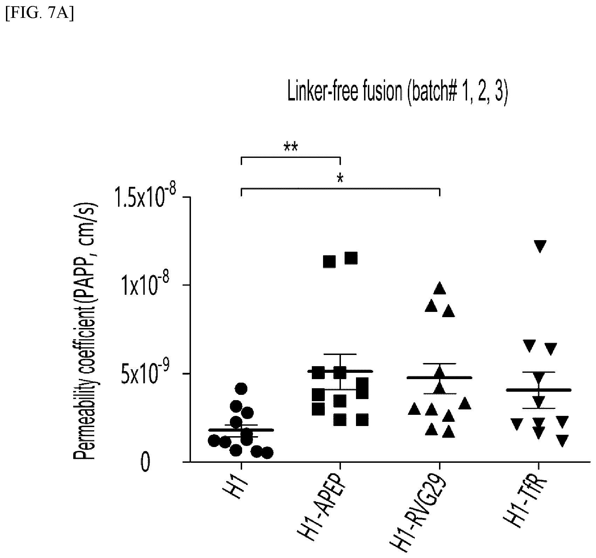

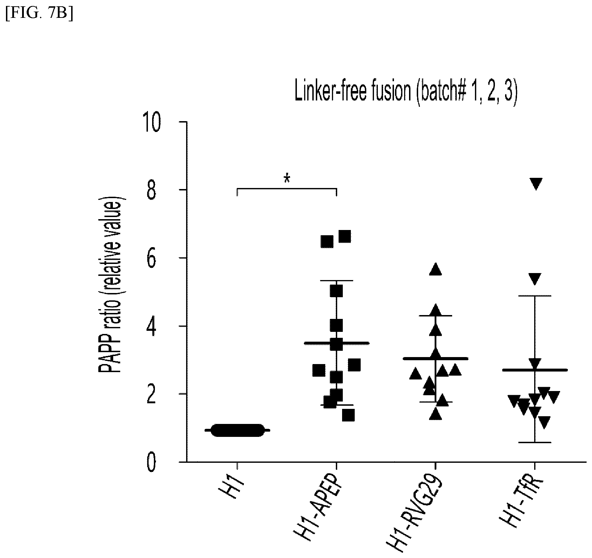

Fig. 7 illustrates graphs showing the total Papp results (including batch# 1 to 3) of the H1 antibody and three antibody-brain penetrating peptide fusions (H1-APEP, H1-RVG29, and H1-TfR) according to embodiments of the present disclosure. Fig. 7A illustrates a graph showing the total Papp results, with average values indicated. Fig. 7B illustrates a graph showing the PAPP ratios (relative values), each obtained by dividing the total Papp result by the Papp value of the H1 antibody, with average values indicated. * indicates $0.01 < P < 0.05$ relative to the H1 antibody, and ** indicates $0.001 < P < 0.01$ relative to the H1 antibody.

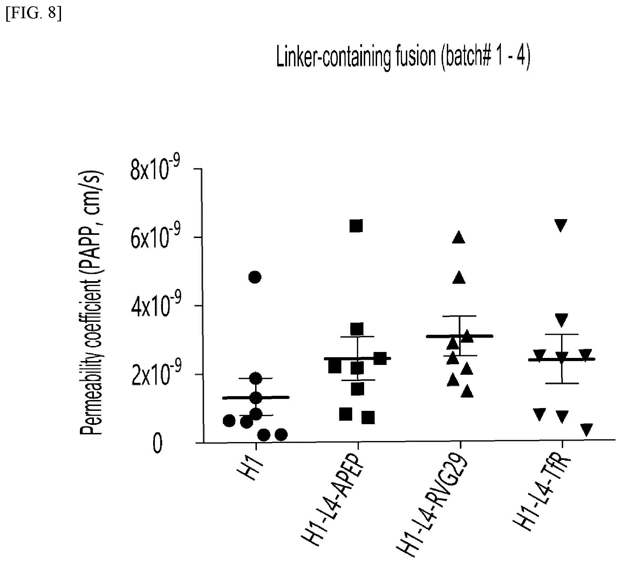

Fig. 8 illustrates a graph showing the total Papp results (including batch# 1 to 4) of the H1 antibody and three antibody-linker-brain penetrating peptide fusions (H1-L4-APEP, H1-L4-RVG29, and H1-L4-TfR) according to embodiments of the present disclosure.

Best Mode for Carrying out Invention

The detailed description of the present disclosure described below will be described with reference to specific drawings regarding specific embodiments in which the present disclosure may be practiced; however, the present disclosure is not limited thereto and is limited only by the appended claims along with any scope equivalent to that described in the claims. It should be understood that the various embodiments/examples of the present disclosure are different from each other but do not need to be mutually exclusive. For example, certain shapes, structures, and features described herein may be altered from one embodiment/example to another, or may be realized in a combination of embodiments/examples, without departing from the spirit and scope of the present disclosure. Unless otherwise indicated, terms used in describing the present disclosure are to be understood in their ordinary meaning and apply to the same terms used herein as well as to the aspect or embodiment of the disclosure for which the term is defined. For purposes of interpreting this specification, the following definition will apply, and whenever appropriate, terms used in the singular will also include the plural and vice versa.

Definition

The "blood-brain barrier" or "BBB" refers to an important biological barrier that protects the brain, which is composed of cerebral vascular endothelial cells and the basement membrane that surrounds and supports them, astrocytes, and pericytes. The blood-brain barrier prevents foreign substances (molecules or small molecules such as pathogens, pigments, drugs, and toxins) from entering the brain through tight junctions of vascular endothelial cells. The blood-brain barrier within the brain, as well as the blood-spinal cord barrier within the spinal cord and the blood-retinal barrier within the retina, are contiguous capillary barriers within the central nervous system and are collectively referred to as the blood-brain barrier or BBB. The blood-spinal cord barrier (choroid plexus) is comprised of ependymal cells rather than capillary endothelial cells.

"Antibody" is used broadly and includes monoclonal antibodies (including full length antibodies) of any isotype, such as IgG, IgM, IgA, IgD, and IgE, polyclonal antibodies, multispecific antibodies (for example, bispecific antibodies), antibody fusions (for example, a fusion of an antibody with a (poly)peptide or a fusion of an antibody with a compound), and antibody fragments (including antigen-binding fragments). As used herein, the prefix "anti-," when in conjunction with an antigen, indicates that the given antibody is reactive with the given antigen. An antibody reactive with a specific antigen may be produced by synthetic and/or recombinant methods, such as, but not limited to, selection of libraries of recombinant antibodies in phage or similar vectors, or by immunizing an animal with the antigen or an antigen-encoding nucleic acid. A typical IgG antibody is comprised of two identical heavy chains and two identical light chains that are joined by disulfide bonds. Each heavy and light chain contains a constant region and a variable region. Each of the heavy chain variable region (HVR) and light chain variable region (LVR) contains three segments, called "complementarity determining regions" ("CDRs") or "hypervariable regions", which are primarily responsible for binding an epitope of an antigen. They are usually referred to as CDR1, CDR2, and CDR3, numbered sequentially from the N-terminus. The more highly conserved portions of the variable regions outside of the CDRs are called "framework regions". As used herein, the antibody may be, for example, an animal antibody, a chimeric antibody, a humanized antibody, or a human antibody.

The term "anti-tau antibody" refers to an antibody that is capable of binding tau with sufficient affinity such that the antibody is useful as a therapeutic agent in targeting tau.

The term "monoclonal antibody" refers to an antibody obtained from a population of substantially homogeneous antibodies, wherein the individual antibodies comprising the population are identical except for possible naturally occurring mutations and/or post-translational modifications (for example, isomerization and amidation) which may be present in minor amounts. Monoclonal antibodies are highly specific and are directed against a single antigenic site. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by a variety of techniques, including the hybridoma method, recombinant DNA methods, phage-display methods, methods utilizing transgenic animals containing all or part of the human immunoglobulin loci, and the like.

The term "antigen-binding fragment" refers to a portion of an antibody, which has specific binding ability to an antigen, or a polypeptide comprising the same. Except where the term "antibody" is understood from the context to specifically exclude "antigen-binding fragments," the terms "antibody" and "antigen-binding fragment" may be used interchangeably, and the "antibody" may be interpreted to include "antigen-binding fragments." Examples of the antigen-binding fragment include, but are not limited to, Fv, Fab, Fab', Fab'-SH, F(ab')2, diabodies, triabodies, tetrabodies, cross-Fab fragments, linear antibodies, single-chain antibody molecules (for example, scFv), and multispecific antibodies formed from antibody fragments and single domain antibodies.

The term "Fc region" as used herein refers to a C-terminal region of an immunoglobulin heavy chain that contains at least a portion of the constant region (for example, CH2, CH3, or CH2 and CH3). The term includes native sequence Fc regions and variant Fc regions.

The term "blood-brain barrier receptor-binding peptide" refers to a peptide that is capable of selectively crossing the blood-brain barrier, which otherwise has restricted transport of substances, by binding to receptors present in the plasma membrane of brain capillary endothelial cells. The blood-brain barrier receptor-binding peptide may be included in brain penetrating peptides (BPPs) or cell penetrating peptides (CPPs) in that it has the ability to cross the endothelial cells of brain capillaries. Therefore, in the present specification, the terms "blood-brain barrier receptor-binding peptide" and "brain penetrating peptide", "cell penetrating peptide", "BPP," or "CPP" may be used interchangeably. In some embodiments, the peptide may comprise or consist of 2 to 50, specifically 8 to 40, more specifically 10 to 30, and even more specifically 12 to 29 amino acid residues.

The term "fusion" may be used interchangeably with "fusion protein" or "fusion polypeptide" and refers to a fusion polypeptide molecule comprising an immunoglobulin molecule and a brain penetrating peptide. In some embodiments, the fusion is a fusion polypeptide comprising an anti-tau antibody and a blood-brain barrier receptor-binding peptide.

The term "linker" may refer to a peptide linker of 1 to 100 amino acids, specifically 2 to 50 amino acids, and more specifically 5 to 30 amino acids in length. The linker may include, but is not limited to, one or more amino acids selected from the group consisting of, for example, Gly, Asn, Ser, Thr, Ala, Asp, and the like. As an example, the linker may be represented by (GGGGS)n, wherein n is the number of repetitions of the unit (GGGGS) and may be 1 to 10, and specifically 1 to 5, in consideration of efficacy of an antibody. As another example, the linker may be a peptide fragment known to be capable of linking antibody fragment domains. For example, the linker may have an amino acid sequence of [GGGGS]$_4$. In an embodiment, preferably, the linker may be an amino acid sequence of GGGGSGGGGSGGGGSGGGGSG.

The term "subject" is used interchangeably with "patient" and may be a mammal, such as a primate (for example, human), a companion animal (for example, dog, cat, and the like), a livestock animal (for example, cow, pig, horse, sheep, goat, and the like), and a laboratory animal (for example, rat, mouse, guinea pig, and the like), in need of prevention or treatment of a tau protein-mediated neurological or neurodegenerative disease. In some implementations, the subject is a human.

The term "treatment" generally means obtaining a desired pharmacological and/or physiological effect. Such an effect has a therapeutic effect in that it partially or completely cures a disease and/or adverse effects caused by the disease. Desirable therapeutic effects include, but are not limited to, preventing occurrence or recurrence of a disease, alleviation of symptoms, diminishment of any direct or indirect pathological consequences of a disease, preventing metastasis, decreasing a rate of disease progression, amelioration or palliation of a disease state, and remission or improved prognosis. Preferably, "treatment" may mean medical intervention for an already manifested disease or disorder.

The term "prevention" refers to prophylactic treatment, that is, a measure or procedure aimed at preventing a disease rather than treating it. The "prevention" means obtaining a desired prophylactic pharmacological and/or physiological effect with a view to partially or completely preventing a disease or symptoms thereof.

The term "administration" means providing a subject with a substance to achieve a prophylactic or therapeutic purpose.

The term "therapeutically effective amount" or "effective amount" refers to an amount of an effective ingredient (substance) that is sufficient to achieve treatment of a disease when administered to a mammal or other subject to treat the disease. The "therapeutically effective amount" or "effective amount" may be determined depending on factors including weight, gender, age, health status and disease severity of a subject (preferably a human), activity of the drug, sensitivity to the drug, time of administration, route of administration and excretion rate, duration of treatment, drugs used concurrently therewith, and other factors well known in the medical field.

Fusion comprising anti-tau antibody or antigen-binding fragment thereof and blood-brain barrier receptor-binding peptide

According to an aspect of the present disclosure, there is provided a fusion comprising an anti-tau antibody or an antigen-binding fragment thereof and a blood-brain barrier receptor-binding peptide. The anti-tau antibody or antigen-binding fragment thereof may be coupled to the blood-brain barrier receptor-binding peptide either directly without a linker or via a linker. Preferably, the anti-tau antibody or antigen-binding fragment thereof may be coupled directly to the blood-brain barrier receptor-binding peptide without a linker.

In some embodiments, the anti-tau antibody or antigen-binding fragment thereof may specifically bind to an acetylated epitope of wild-type tau protein, such as an epitope comprising amino acids at positions 275 to 286 of the wild type tau protein of SEQ ID NO: 25, in which the amino acid at position 280 is acetylated.

In some embodiments, the anti-tau antibody or antigen-binding fragment thereof may comprise a heavy chain variable region (VH) that comprises heavy chain CDR1 comprising or consisting of the amino acid sequence of SEQ ID NO: 2, heavy chain CDR2 comprising or consisting of the amino acid sequence of SEQ ID NO: 4, and heavy chain CDR3 comprising or consisting of the amino acid sequence of SEQ ID NO: 6; and a light chain variable region (VL) that comprises light chain CDR1 comprising or consisting of the amino acid sequence of SEQ ID NO: 10, light chain CDR2 comprising or consisting of the amino acid sequence of SEQ ID NO: 12, and light chain CDR3 comprising or consisting of the amino acid sequence of SEQ ID NO: 14.

In some embodiments, the anti-tau antibody or antigen-binding fragment thereof may comprise a heavy chain variable region comprising an amino acid sequence selected from the group consisting of SEQ ID NO: 8, SEQ ID NO: 27, SEQ ID NO: 29, SEQ ID NO: 31, and SEQ ID NO: 33; and a light chain variable region comprising an amino acid sequence selected from the group consisting of SEQ ID NO: 16, SEQ ID NO: 35, SEQ ID NO: 37, and SEQ ID NO: 39.

In some embodiments, the anti-tau antibody or antigen-binding fragment thereof may be any one selected from the group consisting of:

(1) an anti-tau antibody or an antigen-binding fragment thereof that comprises a heavy chain variable region comprising the amino acid sequence represented by SEQ ID NO: 8 and a light chain variable region comprising the amino acid sequence represented by SEQ ID NO: 16;

(2) an anti-tau antibody or an antigen-binding fragment thereof that comprises a heavy chain variable region comprising the amino acid sequence represented by SEQ ID NO: 27 and a light chain variable region comprising the amino acid sequence represented by SEQ ID NO: 35;

(3) an anti-tau antibody or an antigen-binding fragment thereof that comprises a heavy chain variable region comprising the amino acid sequence represented by SEQ ID NO: 27 and a light chain variable region comprising the amino acid sequence represented by SEQ ID NO: 37;

(4) an anti-tau antibody or an antigen-binding fragment thereof that comprises a heavy chain variable region comprising the amino acid sequence represented by SEQ ID NO: 27 and a light chain variable region comprising the amino acid sequence represented by SEQ ID NO: 39;

(5) an anti-tau antibody or an antigen-binding fragment thereof that comprises a heavy chain variable region comprising the amino acid sequence represented by SEQ ID NO: 29 and a light chain variable region comprising the amino acid sequence represented by SEQ ID NO: 35;

(6) an anti-tau antibody or an antigen-binding fragment thereof that comprises a heavy chain variable region comprising the amino acid sequence represented by SEQ ID NO: 29 and a light chain variable region comprising the amino acid sequence represented by SEQ ID NO: 37;

(7) an anti-tau antibody or an antigen-binding fragment thereof that comprises a heavy chain variable region comprising the amino acid sequence represented by SEQ ID NO: 29 and a light chain variable region comprising the amino acid sequence represented by SEQ ID NO: 39;

(8) an anti-tau antibody or an antigen-binding fragment thereof that comprises a heavy chain variable region comprising the amino acid sequence represented by SEQ ID NO: 31 and a light chain variable region comprising the amino acid sequence represented by SEQ ID NO: 35;

(9) an anti-tau antibody or an antigen-binding fragment thereof that comprises a heavy chain variable region comprising the amino acid sequence represented by SEQ ID NO: 31 and a light chain variable region comprising the amino acid sequence represented by SEQ ID NO: 37;

(10) an anti-tau antibody or an antigen-binding fragment thereof that comprises a heavy chain variable region comprising the amino acid sequence represented by SEQ ID NO: 31 and a light chain variable region comprising the amino acid sequence represented by SEQ ID NO: 39;

(11) an anti-tau antibody or an antigen-binding fragment thereof that comprises a heavy chain variable region comprising the amino acid sequence represented by SEQ ID NO: 33 and a light chain variable region comprising the amino acid sequence represented by SEQ ID NO: 35;

(12) an anti-tau antibody or an antigen-binding fragment thereof that comprises a heavy chain variable region comprising the amino acid sequence represented by SEQ ID NO: 33 and a light chain variable region comprising the amino acid sequence represented by SEQ ID NO: 37; and

(13) an anti-tau antibody or an antigen-binding fragment thereof that comprises a heavy chain variable region comprising the amino acid sequence represented by SEQ ID NO: 33 and a light chain variable region comprising the amino acid sequence represented by SEQ ID NO: 39.

Preferably, the anti-tau antibody or antigen-binding fragment thereof may comprise a heavy chain variable region comprising or consisting of the amino acid sequence of SEQ ID NO: 8 and a light chain variable region comprising or consisting of the amino acid sequence of SEQ ID NO: 16.

In some embodiments, the anti-tau antibody may be a full-length antibody. In addition, the anti-tau antibody may be IgG1, IgG2, IgG3, IgG4, IgA, or IgA2.

For the anti-tau antibody, reference may also be made to Korean Patent No. 10-2196840, the entire disclosure of which is incorporated herein in its entirety.

In some embodiments, the blood-brain barrier receptor-binding peptide may be any one selected from the group consisting of Angiopep-2 (APEP), RVG29, and TfR binding peptide (TfR). The APEP peptide may comprise or consist of the amino acid sequence of SEQ ID NO: 20. The RVG29 peptide may comprise or consist of the amino acid sequence of SEQ ID NO: 22. The TfR binding peptide may comprise or consist of the amino acid sequence of SEQ ID NO: 24. In an embodiment of the present disclosure, it was identified that the anti-tau antibody exhibited excellent blood-brain barrier (BBB) permeability when coupled with the APEP, RVG29, or TfR-binding peptide.

In some embodiments, in the fusion, the anti-tau antibody may be coupled to the blood-brain barrier receptor-binding peptide via a linker. When coupling is achieved via a linker, the linker may be a peptide fragment known to be capable of linking antibody fragment domains, and may be, for example, have an amino acid sequence of [GGGGS]$_4$. In an embodiment, preferably, the linker may be an amino acid sequence of GGGGSGGGGSGGGGSGGGSG. Specifically, the blood-brain barrier receptor-binding peptide may be coupled, via a linker, to Fc region of the anti-tau antibody, for example, the C-terminus of the heavy chain.

The present disclosure is based in part on the surprising discovery that the anti-tau antibody is capable of crossing the blood-brain barrier (BBB) more effectively when coupled to the blood-brain barrier receptor-binding peptide without a linker. Accordingly, in another embodiment, the fusion may be such that the anti-tau antibody is coupled directly to the blood-brain barrier receptor-binding peptide without a linker. For example, the anti-tau antibody may comprise a light chain and a heavy chain, and the brain penetrating peptide may be bound to Fc region of the heavy chain (for example, the C-terminus of the Fc region). In an embodiment of the present disclosure, significantly improved BBB permeability was observed when the APEP, RVG29, or TfR peptide, in particular, the APEP or RVG29 peptide was directly coupled to the anti-tau antibody.

The nucleotide and amino acid sequences for the anti-tau antibody, linker, and brain penetrating peptide, which are included in exemplary fusions of the present disclosure, are as follows.

[Table 1]

| Domain | | DNA sequence (with CDR1, CDR2, and CDR3 shown in bold and underlined in this order) | SEQ ID NO |
|---|---|---|---|
| Heavy chain variable region | Full sequence | gaagtacaacttcaagagtctggcccccggtctcgtcaagccctctcaaacactcagtttg acatgcaccgtatct**ggagacagcatcacaagtggttac**tggaactggattaggcagc cacccggtaagggcttggaatatattggatat**atcagatattctggacgcaca**tactata acccaagtcttaaatctagggtgaccataagcagagatacaagtaagaaccaattctctct caagctttctagcgtcacagccgccgacactgcagtctattattgc**gcttctgtttatttca cttat**tggggtcaaggaactctggtaaccgttagctca | 7 |
| | CDR1 | **ggagacagcatcacaagtggttac** | 1 |
| | CDR2 | **atcagatattctggacgcaca** | 3 |
| | CDR3 | **gcttctgtttatttcacttat** | 5 |

(continued)

| Domain | | DNA sequence (with CDR1, CDR2, and CDR3 shown in bold and underlined in this order) | SEQ ID NO |
|---|---|---|---|
| Light chain variable region | Full sequence | gacgttgtgatgacacaaagccctctgagcctgcctgtgacactgggacagcctgccag catcagctgcaagtctagc**cagagcctgctggactccgacggcaagacctac**ctgaa ctggttccagcagaggcccggacagagcccccaagagactgatcagc**ctggtgtcc**aa gctggatagcggcgtgcccgatagattttctggctctggcagcggcaccgacttcaccct gaagatcagcagagtggaagccgaggacgtgggcgtgtactactgt**tggcagggctc tcacttcccctacacc**tttggccagggcacaaagctggaaatcaag | 15 |
| | CDR1 | **cagagcctgctggactccgacggcaagacctac** | 9 |
| | CDR2 | **ctggtgtcc** | 11 |
| | CDR3 | **tggcaggcctctcacttcectacace** | 13 |
| Linker | | ggcggcggcggctctggaggaggaggcagcggcggcggcggctctggaggaggct ccggc | 17 |
| brain penetrating peptide | APEP | acttttttttatggtggttctcgtggtaaacgtaataattttaaaactgaagaatat | 19 |
| | RVG29 | tacaccatctggatgccagagaaccccccggcctggcaccccatgcgacatcttcacaaa cagccggggcaagagagcctccaatggc | 21 |
| | TfR | acccacaggccacctatgtggagcccagtgtggccc | 23 |

[Table 2]

| Domain | | Amino acid sequence (with CDR1, CDR2, and CDR3 shown in bold and underlined in this order) | SEQ ID NO |
|---|---|---|---|
| Heavy chain variable region | Full sequence | EVQLQESGPGLVKPSQTLSLTCTVS**GDSITSGY**WN WIRQPPGKGLEYIGY**IRYSGRT**YYNPSLKSRVTISR DTSKNQFSLKLSSVTAADTAVYYC**ASVYFTY**WGQ GTLVTVSS | 8 |
| | CDR1 | **GDSITSGY** | 2 |
| | CDR2 | **IRYSGRT** | 4 |
| | CDR3 | **ASVYFTY** | 6 |
| Light chain variable region | Full sequence | DVVMTQSPLSLPVTLGQPASISCKSS**QSLLDSDGKT Y**LNWFQQRPGQSPKRLIS**LVS**KLDSGVPDRFSGSGS GTDFTLKISRVEAEDVGVYYC**WQGSHFPYT**FGQG TKLEIK | 16 |
| | CDR1 | **QSLLDSDGKTY** | 10 |
| | CDR2 | **LVS** | 12 |
| | CDR3 | **WQGSHFPYT** | 14 |

(continued)

| Domain | | Amino acid sequence (with CDR1, CDR2, and CDR3 shown in bold and underlined in this order) | SEQ ID NO |
|---|---|---|---|
| Linker | | GGGGSGGGGSGGGGSGGGGSG | 18 |
| Brain penetrating peptide | APEP | TFFYGGSRGKRNNFKTEEY | 20 |
| | RVG29 | YTIWMPENPRPGTPCDIFTNSRGKRASNG | 22 |
| | TfR | THRPPMWSPVWP | 24 |

The amino acid sequences of exemplary anti-tau antibodies, which may be included in the fusion of the present disclosure, are as shown in Table 3.

[Table 3]

| Antibody | VH amino acid sequence | SEQ ID NO | VL amino acid sequence | SEQ ID NO |
|---|---|---|---|---|
| Antibody 1 | EVQLQESGPGLVKPSQTLSLTCTVSGDSITSGYWNWIRQPPGKGLEYIGYIRYSGRTYYNPSLKSRVTISRDTSKNQFSLKLSSVTAADTAVYYCASVYFTYWGQGTLVTVSS | 8 | DVVMTQSPLSLPVTLGQPASISCKSSQSLLDSDGKTYLNWFQQRPGQSPKRLISLVSKLDSGVPDRFSGSGSGTDFTLKISRVEAEDVGVYYCWQGSHFPYTFGQGTKLEIK | 16 |
| Antibody 2 | EVQLEESGPSLVKPSQTLSLTCSVTGDSITSGYWNWIRKFPGNKLEYMGYIRYSGRTYYNPSLKSRISITRDTSKNQFYLQLISVTTEDTATYYCASVYFTYWGQGTLVTVSS | 27 | DVLMTQTPLTLSVTIGQPASISCKSSQSLLDSDGKTYLNWLLQRPGQSPKRLISLVSKLDSGVPDRFTGSGSGTDFTLKISRVEAEDLGVYYCWQGSHFPYTFGGGTKLEIK | 35 |

(continued)

| Antibody | VH amino acid sequence | SEQ ID NO | VL amino acid sequence | SEQ ID NO |
|---|---|---|---|---|
| Antibody 3 | EVQLEESGPSLVKPSQTLSLTCSVTGDSITSGYWNWIRKFPGNKLEYMGYIRYSGRTYYNPSLKSRISITRDTSKNQFYLQLISVTTEDTATYYCASVYFTYWGQGTLVTVSS | 27 | DVVMTQSPDSLAVSLGERATINCKSSQSLLDSDGKTYLNWYQQKPGQSPKRLISLVSKLDSGVPDRFSGSGSGTDFTLTISSLQAEDVAVYYCWQGSHFPYTFGQGTKLEIK | 37 |
| Antibody 4 | EVQLEESGPSLVKPSQTLSLTCSVTGDSITSGYWNWIRKFPGNKLEYMGYIRYSGRTYYNPSLKSRISITRDTSKNQFYLQLISVTTEDTATYYCASVYFTYWGQGTLVTVSS | 27 | EVVLTQSPGTLSLSPGERATLSCKSSQSLLDSDGKTYLNWYQQKPGQSPKRLISLVSKLDSGIPDRFSGSGSGTDFTLTISRLEPEDFAVYYCWQGSHFPYTFGQGTKLEIK | 39 |
| Antibody 5 | EVQLQQSGPGLVKPSQTLSLTCAVSGDSITSGYWNWIRQSPSRGLEWLGYIRYSGRTYYNPSLKSRITINRDTSKNQFSLQLNSVTPEDTAVYYCASVYFTYWGQGTLVTVSS | 29 | DVLMTQTPLTLSVTIGQPASISCKSSQSLLDSDGKTYLNWLLQRPGQSPKRLISLVSKLDSGVPDRFTGSGSGTDFTLKISRVEAEDLGVYYCWQGSHFPYTFGGGTKLEIK | 35 |

(continued)

| Antibody | VH amino acid sequence | SEQ ID NO | VL amino acid sequence | SEQ ID NO |
|---|---|---|---|---|
| Antibody 6 | EVQLQQSGPGLVKPS QTLSLTCAVSGDSITS GYWNWIRQSPSRGLE WLGYIRYSGRTYYNP SLKSRITINRDTSKNQF SLQLNSVTPEDTAVY YCASVYFTYWGQGTL VTVSS | 29 | DVVMTQSPDSLAVSLGE RATINCKSSQSLLDSDGK TYLNWYQQKPGQSPKRL ISLVSKLDSGVPDRFSGS GSGTDFTLTISSLQAEDV AVYYCWQGSHFPYTFGQ GTKLEIK | 37 |
| Antibody 7 | EVQLQQSGPGLVKPS QTLSLTCAVSGDSITS GYWNWIRQSPSRGLE WLGYIRYSGRTYYNP SLKSRITINRDTSKNQF SLQLNSVTPEDTAVY YCASVYFTYWGQGTL VTVSS | 29 | EVVLTQSPGTLSLSPGER ATLSCKSSQSLLDSDGKT YLNWYQQKPGQSPKRLI SLVSKLDSGIPDRFSGSGS GTDFTLTISRLEPEDFAV YYCWQGSHFPYTFGQGT KLEIK | 39 |
| Antibody 8 | EVTLKESGPTLVKPTQ TLTLTCTVSGDSITSG YWNWIRQPPGKALEY LAYIRYSGRTYYNPSL KSRLTITRDTSKNQVV LTMTNMDPVDTATY YCASVYFTYWGQGTL VTVSS | 31 | DVLMTQTPLTLSVTIGQP ASISCKSSQSLLDSDGKT YLNWLLQRPGQSPKRLIS LVSKLDSGVPDRFTGSGS GTDFTLKISRVEAEDLGV YYCWQGSHFPYTFGGGT KLEIK | 35 |

(continued)

| Antibody | VH amino acid sequence | SEQ ID NO | VL amino acid sequence | SEQ ID NO |
|---|---|---|---|---|
| Antibody 9 | EVTLKESGPTLVKPTQ TLTLTCTVSGDSITSG YWNWIRQPPGKALEY LAYIRYSGRTYYNPSL KSRLTITRDTSKNQVV LTMTNMDPVDTATY YCASVYFTYWGQGTL VTVSS | 31 | DVVMTQSPDSLAVSLGE RATINCKSSQSLLDSDGK TYLNWYQQKPGQSPKRL ISLVSKLDSGVPDRFSGS GSGTDFTLTISSLQAEDV AVYYCWQGSHFPYTFGQ GTKLEIK | 37 |
| Antibody 10 | EVTLKESGPTLVKPTQ TLTLTCTVSGDSITSG YWNWIRQPPGKALEY LAYIRYSGRTYYNPSL KSRLTITRDTSKNQVV LTMTNMDPVDTATY YCASVYFTYWGQGTL VTVSS | 31 | EVVLTQSPGTLSLSPGER ATLSCKSSQSLLDSDGKT YLNWYQQKPGQSPKRLI SLVSKLDSGIPDRFSGSGS GTDFTLTISRLEPEDFAV YYCWQGSHFPYTFGQGT KLEIK | 39 |
| Antibody 11 | EVQLVESGGGLVQPG RSLRLSCTVSGDSITS GYWNWFRQAPGKGL EYVGYIRYSGRTYYN PSLKSRFTISRDTSKNI AYLQMNSLKTEDTAV YYCASVYFTYWGQG TLVTVSS | 33 | DVLMTQTPLTLSVTIGQP ASISCKSSQSLLDSDGKT YLNWLLQRPGQSPKRLIS LVSKLDSGVPDRFTGSGS GTDFTLKISRVEAEDLGV YYCWQGSHFPYTFGGGT KLEIK | 35 |
| Antibody 12 | EVQLVESGGGLVQPG RSLRLSCTVSGDSITS GYWNWFRQAPGKGL EYVGYIRYSGRTYYN | 33 | DVVMTQSPDSLAVSLGE RATINCKSSQSLLDSDGK TYLNWYQQKPGQSPKRL ISLVSKLDSGVPDRFSGS | 37 |

(continued)

| Antibody | VH amino acid sequence | SEQ ID NO | VL amino acid sequence | SEQ ID NO |
|---|---|---|---|---|
|  | PSLKSRFTISRDTSKNI AYLQMNSLKTEDTAV YYCASVYFTYWGQG TLVTVSS |  | GSGTDFTLTISSLQAEDV AVYYCWQGSHFPYTFGQ GTKLEIK |  |
| Antibody 13 | EVQLVESGGGLVQPG RSLRLSCTVSGDSITS GYWNWFRQAPGKGL EYVGYIRYSGRTYYN PSLKSRFTISRDTSKNI AYLQMNSLKTEDTAV YYCASVYFTYWGQG TLVTVSS | 33 | EVVLTQSPGTLSLSPGER ATLSCKSSQSLLDSDGKT YLNWYQQKPGQSPKRLI SLVSKLDSGIPDRFSGSGS GTDFTLTISRLEPEDFAV YYCWQGSHFPYTFGQGT KLEIK | 39 |

In some embodiments, the anti-tau antibody or antigen-binding fragment thereof and the blood-brain barrier receptor-binding peptide may comprise sequences having at least 80%, preferably at least 90%, more preferably at least 95%, and most preferably at least 98% sequence identity to the sequences set forth in Tables 1 and 2.

In another embodiment, the anti-tau antibody or antigen-binding fragment thereof may comprise sequences having at least 80%, preferably at least 90%, more preferably at least 95%, and most preferably at least 98% sequence identity to the sequences set forth in Table 3.

In some embodiments, amino acid sequence variants of the anti-tau antibody or antigen-binding fragment thereof and the blood-brain barrier receptor-binding peptide may be used. For example, it may be desirable for the variants to be such that they improve binding affinity and/or other biological properties of the antibody or peptide. Amino acid sequence variants of the antibody or peptide may be prepared by introduction of appropriate modifications into a nucleotide sequence encoding the molecule or by peptide synthesis. Such modifications include, for example, deletion of residues from, and/or insertion of residues into, and/or substitution of residues within, the corresponding amino acid sequence. Any combination of various changes, including deletion, insertion, and substitution, may be made to arrive at a final construct, provided that the final construct possesses desired properties, such as antigen-binding property. Sites of interest for substitutional mutagenesis include heavy chain variable regions (HVRs) and framework regions (FRs). Conservative substitutions are provided in Table 4 under the heading "Preferred substitution" and are further described below with respect to amino acid side chain classes (1) to (6). Amino acid substitutions may be introduced into the molecules of interest and the products screened for desired activity, for example, retained/improved antigen receptor binding, decreased immunogenicity, or improved ADCC or CDC.

[Table 4]

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |

(continued)

| Original residue | Exemplary substitution | Preferred substitution |
|---|---|---|
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Ile |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties as follows:

(1) hydrophobic: Norleucine, Met, Ala, Val, Leu, Ile;

(2) neutral hydrophilic: Cys, Ser, Thr, Asn, Gln;

(3) acidic: Asp, Glu;

(4) basic: His, Lys, Arg;

(5) residues that influence chain orientation: Gly, Pro;

(6) aromatic: Trp, Tyr, Phe.

Non-conservative substitutions involve exchanging a member of one of these classes for that of another class.

Nucleic acid, vector, host cell, and preparation method

According to still yet another aspect of the present disclosure, there is provided a polynucleotide encoding the fusion, which comprises an anti-tau antibody or an antigen-binding fragment thereof and a blood-brain barrier receptor-binding peptide, as disclosed herein, or heavy chain and/or light chain polypeptides included in the fusion.
In an embodiment, the nucleotide sequence encoding the fusion codes for a light chain variable region, and may comprise a nucleotide sequence that encodes the CDR sequences or the entire variable region sequence as set forth in Table 1, or a sequence having at least 80%, preferably at least 90%, more preferably at least 95%, and most preferably at least 98% sequence identity to the nucleotide sequence.
In an embodiment, the nucleotide sequence encoding the fusion codes for a heavy chain variable region, and may comprise a nucleotide sequence that encodes the CDR sequences or the entire variable region sequence as set forth in Table 1, or a sequence having at least 80%, preferably at least 90%, more preferably at least 95%, and most preferably at least 98% sequence identity to the nucleotide sequence.
In an embodiment, the nucleotide sequence encoding the fusion codes for a heavy chain variable region and a blood-brain barrier receptor-binding peptide, and may comprise a nucleotide sequence encoding the CDR sequences or the entire variable region sequence as set forth in Table 1 and a nucleotide sequence encoding the blood-brain barrier receptor-binding peptide, or may comprise sequences having at least 80%, preferably at least 90%, more preferably at least 95%, and most preferably at least 98% sequence identity to the nucleotide sequences.

In an embodiment, in a case where the blood-brain barrier receptor-binding peptide is linked directly or via a linker to a heavy chain of the antibody, there may be provided a nucleic acid that encodes the blood-brain barrier receptor-binding peptide, optionally the linker, and a heavy chain polypeptide comprising a heavy chain variable region of the antibody.

According to still yet another aspect of the present disclosure, there is provided a vector comprising the polynucleotide sequence disclosed herein. The "vector", unless otherwise specified, refers to a material capable of transporting a genetic material into a cell. In addition, in the present disclosure, the "vector" may be an "expression vector" comprising essential regulatory elements operatively-linked to an inserted gene so that the gene is normally expressed. In a case where the fusion comprising the anti-tau antibody includes two separate polypeptides, the nucleotide sequences encoding the two polypeptides may be cloned into the same vector or into separate vectors. One type of vector is a "plasmid," which refers to a circular double-stranded DNA loop into which additional DNA segments can be inserted. Another type of vector is a viral vector, wherein virally-derived DNA or RNA sequences are present in the vector for packaging into a virus. Certain vectors are capable of autonomous replication in a host cell into which they are introduced (for example, bacterial vectors having a bacterial origin of replication and episomal mammalian vectors). Other vectors (for example, non-episomal mammalian vectors) may be integrated into the genome of a host cell upon introduction into the host cell, and thereby are replicated along with the host genome. Common expression vectors of utility in recombinant DNA techniques are often in the form of plasmids. In the present specification, "plasmid" and "vector" may be used interchangeably, as the plasmid is the most commonly used form of vector.

In an embodiment, the vector may be a viral vector. Specifically, the viral vector may be at least one selected from the group consisting of a retrovirus vector, a lentivirus vector, an adenovirus vector, an adeno-associated virus vector, a vaccinia virus vector, a poxvirus vector, a herpes simplex virus vector, and a phagemid vector.

In another embodiment, the vector may be a non-viral vector. Specifically, the non-viral vector may be at least one selected from the group consisting of, but not limited to, plasmid, naked DNA, DNA complex, mRNA (transcript), and amplicon. For example, the plasmid may be selected from the group consisting of pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19.

According to still yet another aspect of the present disclosure, there is provided a host cell comprising the polynucleotide or expression vector disclosed herein. In some embodiments, the host cell may produce a fusion. Any host cell known in the art that allows stable and continuous cloning and expression of the polynucleotide or expression vector may be used. Suitable prokaryotic host cells include Escherichia coli, Bacillus species strains, such as Bacillus subtilis and B. thuringensis, enteric bacteria and strains, such as Salmonella typhimurium, Serratia marcescens, and various Pseudomonas species. Suitable eukaryotic host cells for transformation include yeast (for example, Saccharomyces cerevisiae), insect cells, plant cells, and animal cells (for example, Sp2/0, Chinese hamster ovary (CHO) K1, CHO DG44, PER.C6, W138, BHK, COS-7, 293, HepG2, Huh7, 3T3, RIN, and MDCK cell lines). In addition, the "host cell" is used to refer to a transformed cell or a cell that has been transformed with a nucleotide sequence and is capable of expressing the selected gene of interest. The term includes a progeny of an original parent cell, whether or not the progeny is identical in morphology or genetic make-up to the parent cell, so long as a selected gene is present.

In addition, in a case of introducing an expression vector into a host cell, $CaCl_2$ precipitation method, Hanahan method with increased efficiency by using DMSO (dimethyl sulfoxide) as a reducing agent in $CaCl_2$ precipitation method, electroporation, calcium phosphate precipitation method, protoplast fusion method, stirring method using silicon carbide fiber, agrobacterium-mediated transformation method, transformation method using PEG, dextran sulfate, lipofectamine, drying/inhibition-mediated transformation method, and the like may be used; however, the methods are not limited thereto.

According to still yet another aspect of the present disclosure, there is provided a method for producing the fusion disclosed herein, comprising culturing a host cell. In some embodiments, the method for producing the fusion, which comprises culturing a host cell, may comprise: (i) culturing a host cell to obtain a culture (for example, a culture comprising the fusion); and (ii) recovering the fusion from the culture.

The culturing of a host cell may be carried out using a suitable medium and culture conditions known in the art. Specifically, the culturing may be performed in a batch process, or continuously in a fed-batch or repeated fed-batch process.

The recovering of the fusion from the culture may be performed by methods known in the art. The recovering methods may include, but are not limited to, centrifugation, filtration, extraction, spraying, drying, precipitation, crystallization, electrophoresis, fractional precipitation (for example, ammonium sulfate precipitation), chromatography (for example, ion exchange, affinity, hydrophobic, and size exclusion), and the like.

In addition, the fusions disclosed herein may be prepared by any known method, for example, conventional synthetic methods for protein synthesis, recombinant DNA methods, and the like.

Pharmaceutical composition

According to still yet another aspect of the present disclosure, there is provided a pharmaceutical composition comprising the fusion, which comprises an anti-tau antibody or an antigen-binding fragment thereof and a blood-brain barrier receptor-binding peptide, as disclosed herein. The fusion may be included in the composition in a prophylactic or therapeutically effective amount. The pharmaceutical composition may be administered to a subject for inhibiting aggregation of abnormal tau protein, or for preventing or treating a neurodegenerative disease.

In some embodiments, the neurodegenerative disease may be a degenerative brain disease. In addition, the neurodegenerative disease may be a tau protein-mediated neurological disease. The neurodegenerative disease may be selected from the group consisting of, but not limited to, tauopathy, primary age-related tauopathy, chronic traumatic encephalopathy, progressive supranuclear palsy, corticobasal degeneration, frontotemporal dementia, Lytico-Bodig disease, Parkinson's disease, subacute sclerosing meningitis, lead encephalopathy, tuberous sclerosis, ganglioglioma, gangliocytoma, meningioangiomatosis, subacute sclerosing panencephalitis, Hallervorden-Spatz disease, and lipofuscinosis.

The tauopathy may be selected from the group consisting of, but not limited to, Alzheimer's disease (AD), progressive supranuclear palsy, corticobasal degeneration, Pick's disease, a group of related disorders collectively referred to as frontotemporal dementia (FTDP-17) with Parkinsonism linked to chromosome 17, amyotrophic lateral sclerosis, Creutzfeldt-Jakob disease, dementia pugilistica, Gerstmann-Straussler-Scheinker disease, Lewy body disease, chronic traumatic encephalopathy, and Huntington's disease.

To prepare the pharmaceutical composition, the fusion may be mixed with a pharmaceutically acceptable carrier and/or excipient. The pharmaceutical compositions may be prepared in the form of lyophilized preparations or aqueous solutions. See, for example, Remington's Pharmaceutical Sciences and U.S. Pharmacopeia: National Formulary, Mack Publishing Company, Easton, PA (1984). The acceptable carriers and/or excipients (including stabilizers) are non-toxic to subjects at the dosages and concentrations used and may include, but are not limited to, buffers (for example, phosphate, citrate, or other organic acids); antioxidants (for example, ascorbic acid or methionine); preservatives (for example, octadecyldimethylbenzyl ammonium chloride; hexamethonium chloride; benzalkonium chloride, benzethonium chloride; phenol, butyl, or benzyl alcohol; alkyl parabens such as methyl or propyl paraben; catechol; resorcinol; cyclohexanol; 3-pentanol; and m-cresol); low molecular weight (about 10 or fewer residues) polypeptides; proteins (for example, serum albumin, gelatin, or immunoglobulin); hydrophilic polymers (for example, polyvinylpyrrolidone); amino acids (for example, glycine, glutamine, asparagine, histidine, arginine, or lysine); monosaccharides, disaccharides, and other carbohydrates such as glucose, mannose, or dextrin; chelating agents (for example, EDTA); sugars (for example, sucrose, mannitol, trehalose, or sorbitol); salt-forming counterions (for example, sodium); metal complexes (for example, Zn-protein complexes); and/or nonionic surfactants (for example, TWEEN®, PLURONICS®, or polyethylene glycol (PEG)).

The pharmaceutical composition may be administered orally or parenterally depending on a desired method. If desired for local treatment, intralesional administration may be used. Parenteral administration may include, but is not limited to, intramuscular, intravenous, intraarterial, intraperitoneal, intrapulmonary, transdermal, subcutaneous, intradural, intrarectal, intravesical, intravaginal, intraarticular, intranasal, intrathecal, intracerebral, or intracerebroventricular administration. Dosing may be by any suitable route, for example, by injections, such as intravenous or subcutaneous injections, depending in part on whether the administration is brief or chronic. Various dosing schedules may be contemplated, including, but not limited to, single or multiple administrations over various time-points, bolus administration, and pulse infusion.

The pharmaceutical composition may be formulated into a suitable form known in the art depending on its route of administration.

Prophylactic or therapeutic method

According to still yet another aspect of the present disclosure, there is provided a method for preventing or treating a neurodegenerative disease, comprising administering to a subject in need thereof the fusion, which comprises an anti-tau antibody or an antigen-binding fragment thereof and a blood-brain barrier receptor-binding peptide, as disclosed herein. For the neurodegenerative disease, see the definition as described above.

According to still yet another aspect of the present disclosure, there is provided a method for inhibiting abnormal aggregation of tau protein in a subject, comprising administering to the subject the fusion disclosed herein.

In some embodiments, the method for inhibiting abnormal aggregation of tau protein may be provided by administration of a pharmaceutical composition comprising a fusion in which an anti-tau antibody or an antigen-binding fragment thereof is coupled to a blood-brain barrier receptor-binding peptide either directly without a linker or via a linker. Preferably, the fusion may be provided by administration of a pharmaceutical composition comprising a fusion in which an anti-tau antibody or an antigen-binding fragment thereof and a blood-brain barrier receptor-binding peptide are directly linked to each other without a linker.

In some embodiments, the fusion of the present disclosure may be delivered effectively across the blood-brain barrier using various suitable compositions and methods described herein or known in the art.

In some embodiments, the fusion of the present disclosure may be administered in combination with one or more other agents effective for prevention or treatment of a neurodegenerative disease. The other agents include, but are not limited to, any agent capable of improving or ameliorating a neurodegenerative disease, such as compounds, gene therapeutics, and proteins (including antibodies).

**Mode for Carrying out Invention**

Hereinafter, the present disclosure will be described in more detail by way of the following examples. The following examples are presented to help understand the present disclosure. These examples are not intended to limit a scope of the present disclosure in any way and should not be construed as limiting the present disclosure.

<u>Examples</u>

**Example 1. Production of fusion comprising anti-tau antibody and blood-brain barrier receptor-binding peptide**

**Example 1.1. Construction of expression vector**

A method for producing the fusion, which comprises an anti-tau antibody and a blood-brain barrier receptor-binding peptide, as shown in Figs. 1A and 1B is as follows. First, a request was made to GenScript, Inc. for cloning of the gene sequences of the three respective blood-brain barrier receptor-binding peptides (APEP, RVG29, and TfR). The vector used for cloning was pcDNA3.1(+). For the nucleotide and amino acid sequence of the three peptides, see Table 5. For the nucleotide and amino acid sequences of the anti-tau antibody, see Tables 1 and 2. Hereinafter, the blood-brain barrier receptor-binding peptide is indicated by a brain penetrating peptide (BPP).

[Table 5]

| Domain | | DNA sequence | SEQ ID NO |
|---|---|---|---|
| Brain penetrating peptide | APEP | acttttttttatggtggttctcgtggtaaacgtaataattttaaaactgaagaatat | 19 |
| | RVG29 | tacaccatctggatgccagagaaccccccggcctggcaccccatgcgacatctt cacaaacagccggggcaagagagcctccaatggc | 21 |
| | TfR | acccacaggccacctatgtggagcccagtgtggccc | 23 |
| Domain | | Amino acid sequence | SEQ ID NO |
| Brain penetrating peptide | APEP | TFFYGGSRGKRNNFKTEEY | 20 |
| | RVG29 | YTIWMPENPRPGTPCDIFTNSRGKRASNG | 22 |
| | TfR | THRPPMWSPVWP | 24 |

The nucleotide sequence of each of the three brain penetrating peptides was inserted into the vector using the restriction enzymes XhoI and XbaI, and the vector thus constructed is shown in Fig. 2. Next, the primers for linker insertion were prepared as shown in Table 6 and used to amplify each peptide nucleotide sequence and a portion of the vector by PCR. The nucleotide sequence was inserted into the XhoI-XbaI sites of the pcDNA3.1(+) vector. TfR was exceptionally synthesized with a linker sequence ([GGGGS]$_4$), and cloning for removal of the linker sequence was performed.

[Table 6]

| Amplification target | Direction | Sequence (5'→3') |
|---|---|---|
| [GGGGS]4-APEP | Forward | AAA ACT CGA GGG CGG CGG CGG CTC TGG AGG AGG AGG CAG CGG CGG CGG CGG CTC TGG AGG AGG CTC CGG CAC TTT TTT TTA TGG TGG TTC TCG TGG TAA ACG |
| [GGGGS]4-RVG29 | Forward | AAA ACT CGA GGG AGG AGG AGG CAG CGG GGG AGG AGG CAG CGG AGG AGG AGG CAG CGG CGG CGG CTC CGG CTA CAC CAT CTG GAT GCC AGA GAA CCC |
| TfR | Forward | AAA ACT CGA GAC CCA CAG GCC ACC TAT G |
| Common to BPPs (part of pcDNA3.1 vector) | Reverse | AAG CTC CCG GGA GCT TTT GCA AAG C |

For the fusions, a total of six vectors were constructed using the three brain penetrating peptides depending on the presence or absence of the linker. Then, the heavy chain gene of the anti-tau antibody (H1) was inserted upstream of the brain penetrating peptide (or linker-brain penetrating peptide) using the restriction enzymes BamHI and XhoI to construct an expression vector for the fusion comprising the antibody and the brain penetrating peptide (Fig. 3A). In addition, the light chain gene was inserted into the pcDNA3.1(+) vector using the restriction enzymes HindIII and XbaI to construct an expression vector (Fig. 3B).

Meanwhile, for the anti-tau antibody (H1), the heavy chain gene was inserted into the pcDNA3.1(+) vector using the restriction enzymes BamHI and XhoI, and the light chain gene was inserted into the pcDNA3.1(+) vector using the restriction enzymes HindIII and XbaI, thereby constructing expression vectors that do not comprise a brain penetrating peptide (or linker-brain penetrating peptide) sequence.

**Example 1.2. Production of recombinant fusions by culturing Expi293F cells**

The recombinant vectors of Example 1.1 were introduced into Expi HEK293F cells to produce fusions comprising an antibody and a peptide.

First, the Expi HEK293F cells were subcultured by incubation in a shaking incubator (37°C, 8% $CO_2$, 120 rpm) for 2 to 3 days so that the cells reached a cell count of $5\times10^5$ cells/mL. The culture medium used was FreeStyle™ 293 Expression Medium supplemented with 1X Antibiotic-Antimycotic.

Next, for gene introduction, 200 μg of plasmid vector was added to 10 mL of 150 mM NaCl and mixed thoroughly. Then, 400 μL of PEI (polyethyleneimine hydrochloride, 1 mg/mL) was added thereto (based on 100 mL of the culture). After 15 minutes at room temperature, the solution was added dropwise onto the cell culture. Then, the cells were cultured continuously for 5 to 7 days. The number of cells for gene introduction was adjusted to meet $2\times10^6$ cells/mL based on the cell culture.

The cell culture was centrifuged at 13,000 rpm for 15 minutes at 4°C. Only the supernatant, from which the cells were removed, was taken, filtered through a 0.45 μm filter, and then purified by flowing through the MabSelect SURE column (Cytiva, HiTrap Protein G) equilibrated with 1X PBS. To elute the antibody bound to the column, 0.1 M glycine-HCl, pH 2.7 buffer was flowed through the column while collecting the eluted antibody. To neutralize the pH, 1 M Tris-HCl, pH 9.0 buffer was added to restore the pH to between 7 and 8, followed by final buffer exchange to 1X PBS.

Through the above-described procedure, the fusions comprising an antibody and a brain penetrating peptide were obtained, and the resulting antibody and fusions are described in Table 7.

[Table 7]

| No. | Category | Types of antibody and fusion |
|---|---|---|
| 1 | Antibody | H1 (human anti-tau antibody) |
| 2 | Antibody + Brain penetrating peptide | H1 + Angiopep-2 (APEP) |
| 3 | | H1 + RVG29 |
| 4 | | H1 + TfR binding peptide (TfR) |
| 5 | Antibody + Linker + Brain penetrating peptide | H1 + Linker (L4) + Angiopep-2 (APEP) |
| 6 | | H1 + Linker (L4) + RVG29 |
| 7 | | H1 + Linker (L4) + TfR binding peptide (TfR) |

## Example 2. Production of blood-brain barrier (BBB) model using induced pluripotent stem cells (iPSCs)

In order to check BBB permeability of the fusions according to embodiments of the present disclosure, a BBB model was established as follows using differentiation of human brain microvascular endothelial cells with reference to Lippmann, E., Azarin, S., Kay, J. et al. Derivation of blood-brain barrier endothelial cells from human pluripotent stem cells. Nat Biotechnol 30, 783-791 (2012).

### Example 2.1. Induction of differentiation into human brain microvascular endothelial cells (iBMECs)

Monoculture

To create a blood-brain barrier (BBB) model, induced pluripotent stem cells (iPSCs) (WiCell Research Institute Inc, IMR90-4) were induced to differentiate into human brain microvascular endothelial cells (BMECs).

First, iPSCs were seeded onto a Matrigel-coated 6-well plate at $3 \times 10^5$ cells/well, and then culture was performed by adding mTeSR (containing 10 $\mu$M Y27, total 2 mL/well). Every 24 hours after seeding, the entire medium was replaced with mTeSR1 (without Y27). When the cells reached a cell count of $2.5 \times 10^5$ to $4 \times 10^5$ cells/well (preferably $3 \times 10^5$ cells/well), the medium was replaced with 2 mL of UM (DMEM/F12 medium (containing 15 mM HEPES), 20% KOSR, 1% NEAA, 0.5% glutamax, and $\beta$-ME) medium per well to induce differentiation into induced BMECs (iBMECs) (UM phase, D0). For 1 to 5 days (D1 to D5) after the initiation of induction of differentiation, the medium was replaced daily (2 to 3 mL/well) with UM (containing 1% B27); and on day 6, the medium was replaced with EC (containing hESFM medium, 2% B27 and 0.02% bFGF) containing 0.1% retinoic acid (RA).

On day 7 after the induction of differentiation, a transwell (consisting of a plate and an insert) for subculturing iBMECs was coated with an ECM solution (collagen: fibronectin: water = 4:1:5) as follows. The membrane filter in the transwell insert was coated with an ECM solution at 37°C for at least 4 hours or up to 24 hours prior to subculture. Here, the plate was coated only when performing triple culture.

On day 8 after the induction of differentiation, the ECM solution was removed from the transwell insert, and then the insert was allowed to dry. The cultured iBMECs were harvested using Accutase and hESFM medium, resuspended in an appropriate volume of EC medium (containing 0.1% RA), and seeded into the transwell insert at $1 \times 10^6$ cells/cm$^2$. The insert seeded with iBMECs were placed on the plate, and the plate was shaken back and forth or side to side to prevent the cells from clumping together so that they were evenly distributed. The plate was then incubated in a 37°C incubator, and the medium was replaced with EC (containing hESFM medium, 2% B27) every 24 hours.

A schematic diagram of the above-described differentiation process is shown in Fig. 4.

Triple culture

Triple culture was conducted in the same manner as the monoculture of iBMECs until day 6 after the induction of differentiation. On day 7, astrocytes (ScienceCell, Catalog #1800) and pericytes (ScienceCell, Catalog #1200) were seeded on a 12-transwell plate using the following two methods. The astrocytes ($4.4 \times 10^4$ cells) and pericytes ($4.4 \times 10^4$ cells) were cultured at a 1:1 ratio in pericyte medium, or the astrocytes ($1.5 \times 10^4$ cells) and pericytes ($3 \times 10^4$ cells) were cultured at a 1:2 ratio in 500 $\mu$L of pericyte medium.

On day 8 after the induction of differentiation, the medium in the plate was replaced with endothelial cell medium (containing 10 $\mu$M RA), and iBMECs were seeded into the transwell insert in the same manner as the monoculture to prepare a BBB model comprising three types of cells (endothelial cells, astrocytes, and pericytes).

For the monoculture or triple culture model seeded on the transwell, medium replacement was conducted in such a way that the medium in the plate was removed and then the medium in the insert was removed. Addition of hESFM medium (containing 2% B27) was conducted in such a way that the medium was added to the insert and then the medium was added to the plate.

**Example 2.2. Performance evaluation of BBB model**

To verify performance of iBMECs differentiated in the transwell as a BBB model, immunostaining was performed on the iBMECs and expression of tight junction markers therein was observed by fluorescence microscopy. In addition, the formation of tight junctions was verified by measuring transendothelial electrical resistance (TEER) values of the transwell in which iBMECs were differentiated and by assessing permeability to low-molecular-weight substances.

Identification of expression of cell tight junction markers and BBB-associated proteins

On days 6 to 10 after the initiation of differentiation into iBMECs, to identify differentiation into iBMECs and formation of BBB, marker proteins constituting the BBB were immunostained and observed under a fluorescence microscope (Nikon, ECLIPSE Ti). As shown in Fig. 5, it was identified that the differentiated iBMECs cells exhibited expression of occludin, claudin5, and ZO-1 which are tight junction and adhesion junction markers, and expression of glut1, TfR, and LRP1 which are transporter proteins expressed in the BBB.

Measurement of TEER values and identification of permeability to low-molecular-weight substances

TEER values were measured on days 9 to 11 after the initiation of induction of differentiation into iBMECs, and the measurement method was as follows. First, the transwell was taken out of the incubator and was left to stand at room temperature for 20 to 30 minutes to ensure that its resistance value did not fluctuate. Next, a measuring rod was placed in the middle of a gap between the plate wall and the insert, and then the TEER value was measured.

To assess permeability of substances to iBMECs, TEER values were measured in transwells seeded with iBMECs to initially identify the proper formation of tight junctions. To a total of five transwell inserts with differentiated iBMECs and a control (empty transwell) was added 3 kDa dextran (labeled with Cascade Blue) at a concentration of 50 μg/mL, and permeation was allowed to occur for 24 hours. Then, the amount of dextran was measured in both the plate and the insert. As a result, as shown in Table 8, for the control, dextran was detected in the plate starting from 1 hour after addition, and 50% or more of the total dextran was detected in the plate over 24 hours. On the other hand, for the transwells in which iBMECs were differentiated, it was identified that no dextran had escaped into the plates, which verifies that the tight junctions were properly formed enough to prevent passage of low-molecular-weight substances.

[Table 8]

| Concentration of 3 kDa dextran (μg /mL) | | Well 1 | Well 2 | Well 3 | Well 4 | Well 5 | Control |
|---|---|---|---|---|---|---|---|
| Insert | | 30.8 | 36.7 | 30.7 | 38.3 | 34.2 | 12.9 |
| Plate | 1 hr later | 0 | 0 | 0 | 0 | 0 | 0.9 |
| | 2 hrs later | 0 | 0 | 0 | 0 | 0 | 1.0 |
| | 3 hrs later | 0 | 0 | 0 | 0 | 0 | 1.1 |
| | 4 hrs later | 0 | 0 | 0 | 0 | 0 | 1.7 |
| | 6 hrs later | 0 | 0 | 0 | 0 | 0 | 3.0 |
| | 24 hrs later | 0 | 0 | 0 | 0 | 0 | 7.9 |
| Total | | 30.8 | 36.7 | 30.7 | 38.3 | 34.2 | 28.5 |

**Example 3. Identification of BBB permeability of fusions**

On day 9 to day 10 after the induction of differentiation into iBMECs, TEER values of the transwells were measured according to the TEER value measurement method of Example 2.2. Then, the one antibody and six fusions produced in Example 1 were administered to the transwell inserts, and BBB permeability of the antibody and fusions was measured by measurement of permeability coefficient (Papp) and enzyme-linked immunosorbent assay (ELISA).

Meanwhile, after administration of the antibody and fusions, the TEER value of each transwell was measured to check the average value. As a result, it was identified that the average value was approximately 4000 $\Omega \times cm^2$ after administration of the antibody and fusions (Fig. 6), which was similar to that before the administration. Therefore, it was identified that the administration of the antibody and fusions did not affect performance of the BBB model and did not result in any variation between the samples.

**Example 3.1. Measurement of permeability coefficient (Papp)**

The permeability coefficient (Papp) is an absolute value that represents the rate at which a substance passes through per unit area and per unit time. Papp is a value that can be compared regardless of the surface area of transwell and time used in an experiment, and can be expressed by the following equation.

$$Papp = (dQ/dt) / (C0 \times A)$$

Here, dQ/dt represents an amount of a substance that escapes into the plate (basal) region per unit time; A represents an area of the transwell insert ($cm^2$); and C0 represents an initial concentration of a sample introduced into the insert (apical) region of the transwell.

0.5 mL of hESFM (containing 2% B27) medium was prepared in a 1.5 mL tube, and 7 types of antibody and fusions (25 $\mu$g/mL) were each added thereto to prepare antibody-containing solutions. 0.4 mL of the medium was removed from the insert region of the transwell, and then 0.4 mL of 0.5 mL of the antibody-containing solution was added thereto. 24 hours after administration of the antibody-containing solution (D11), 1.5 mL of medium in the plate region and 0.5 mL of medium from the upper chamber were collected for ELISA analysis. The dQ/dt value was calculated based on the results obtained by ELISA analysis. The area of the well used, either from a 12-well transwell (insert: 1.12 $cm^2$, plate: 3.5 $cm^2$) or a 24-well transwell (insert: 0.33 $cm^2$, plate: 2 $cm^2$), was substituted for the variable A; and 0.1 mL of the antibody-containing solution, which remained after addition to the transwell insert, was used to measure the C0 value.

**Example 3.2. Enzyme-linked immunosorbent assay (ELISA)**

The ELISA analysis method for the antibody and fusions, which permeated from the insert area to the plate region, is as follows.

Pep1 was diluted 1:2000 (0.83 mg/mL) using capture buffer (50 mM $NaHCO_3$ and distilled water, pH 9.6). 50 $\mu$L of the diluted solution was added to the plate and incubation was performed (37°C, 600 rpm, 2 hours) for coating. Then, 100 $\mu$L of blocking buffer (100 mM Tris, 0.1% Tween20, distilled water, and 2.5% Casein, pH 7.6) was added to each well, and incubation was performed (37°C, 600 rpm, 2 hours).

Next, using the standard dilution analysis (STD) technique, the standard solution or sample was diluted in the order of 20, 10, 5, 2.5, 1.25, 0.625, 0.3125, and 0 (ng/mL). 50 $\mu$L of the standard solution or sample was loaded into each well and incubation was performed (37°C, 600 rpm, 1 hour).

50 $\mu$L of the antibody-containing solution (peroxidase-labeled human IgG, 1:2000) (Sigma-Aldrich, AP309P) was added to each well and incubation was performed (37°C, 600 rpm, 1 hour). Then, 50 $\mu$L of substrate reagent (R&D Systems, #DY999) was added to each well and incubation was performed at room temperature for 5 minutes. Subsequently, 50 $\mu$L of stop solution (R&D Systems, #DY994) was added to terminate the reaction, and the absorbance was measured using a spectrometer (Infinite F50) (plate shaking for 10 seconds/measurement wavelength of 450 nm/reference wavelength of 620 nm).

Washing was performed four times in such a manner that each well was filled with wash buffer (PBS containing 0.05% Tween 20) and then the buffer was discarded between the steps except for the step of adding the stop solution after the reaction with substrate reagent.

**Example 3.3. BBB permeability measurement results for fusions**

The monoculture BBB model is composed only of vascular endothelial cells; however, the triple culture BBB model takes a form that comprises not only vascular endothelial cells but also astrocytes and pericytes, which is more similar to the microenvironment of the human blood-brain barrier. For the antibody-brain penetrating peptide fusions (without linker) and antibody-linker-brain penetrating peptide fusions (with linker), their permeability was verified in the monoculture BBB model. Then, for the antibody-brain penetrating peptide fusions that showed statistically significant results, their permeability was also verified in the triple culture BBB model, and the final data were presented as a total result combining the results from both models. The measurements were conducted in a total of four batches based on the production date, producer, concentration, and assay method for the antibody and fusions; and the measurements were conducted either separately for each batch or in the form of mixed batches (Figs. 7 and 8).

Antibody-brain penetrating peptide fusions (without linker)

The overall average Papp values of the H1 antibody and three antibody-brain penetrating peptide fusions (H1-APEP, H1-RVG29, and H1-TfR) in the monoculture BBB model were divided by that of H1 to express ratios for the respective fusions. As a result, all three fusions exhibited higher permeability than the H1 antibody. In addition, the same method was used to express the permeability thereof in the triple culture BBB model. As a result, all three fusions exhibited higher permeability than the H1 antibody (Table 9). Referring to the total results combining the permeability results for monoculture and triple culture, H1-APEP, H1-RVG29, and H1-TfR showed 2.9-fold, 2.7-fold, and 2.3-fold higher permeability, respectively, than the H1 antibody. The total results for the H1 antibody and three antibody-brain penetrating peptide fusions are shown in Fig. 7A, with the Papp values represented on the vertical axis (* indicates 0.01 < P < 0.05, and ** indicates 0.001 < P < 0.01).

[Table 9]

| Average value/average value of H1 | H1 | H1-APEP | H1-RVG29 | H1-TfR |
|---|---|---|---|---|
| Total | 1.0 | 2.9 | 2.7 | 2.3 |
| Monoculture | 1.0 | 3.0 | 2.9 | 2.3 |
| Triple culture | 1.0 | 3.6 | 2.9 | 2.9 |

The results obtained by statistically processing the values, which were calculated by dividing the respective Papp values of the three fusions by that of the H1 antibody also demonstrated that all three fusions exhibited higher permeability than the H1 antibody in the BBB model. Specifically, H1-APEP, H1-RVG29, and H1-TfR showed 3.54-fold, 3.04-fold, and 2.74-fold higher permeability, respectively, than the H1 antibody (Fig. 7B).

Antibody-linker-brain penetrating peptide fusions (with linker)

The overall average Papp values of the H1 antibody and the three antibody-linker-brain penetrating peptide fusions (H1-L4-APEP, H1-L4-RVG29, and H1-L4-TfR) in the monoculture BBB model were divided by that of H1 to express ratios for the respective fusions. As a result, all three fusions exhibited slightly higher permeability (1.9-fold for H1-L4-APEP, 2.4-fold for H1-L4-RVG29, and 1.8-fold for H1-L4-TfR) than the H1 antibody; however, their permeability was lower than that of the fusions without a linker. Here, L4 stands for a linker.

The results obtained by dividing the average Papp values of the fusions by that of H1 are shown in Table 10. For the antibody-linker-brain penetrating peptide fusions, the results from the monoculture represent the total results. The total results (for the monoculture) for the H1 antibody and three antibody-linker-brain penetrating peptide fusions are shown in Fig. 8, with the Papp values represented on the vertical axis.

[Table 10]

| Average value/average value of H1 | H1 | H1-L4-APEP | H1-L4-RVG29 | H1-L4-TfR |
|---|---|---|---|---|
| Total | 1.0 | 1.9 | 2.4 | 1.8 |
| Monoculture | 1.0 | 1.9 | 2.4 | 1.8 |

**Statistical analysis**

Statistical analysis was performed using GraphPad software (GraphPad Software, San Diego, CA, USA). Data were analyzed using one-way ANOVA and Kruskal-Wallis test (nonparametric analysis). In addition, the significance of difference between the group of H1 antibody alone and the group of H1 antibody fused with three brain penetrating peptides (APEP, RVG29, and TfR) was analyzed using Dunn's multiple comparison (*: 0.01 < P < 0.05, **: 0.001 < P < 0.01, ***: P < 0.001, ns: P > 0.05).

**Sequence Listing Free Text**

The sequence information for skipped sequences (fewer than 10 defined nucleotides: SEQ ID NO: 11; and fewer than 4 defined amino acids: SEQ ID NO: 12) in the electronic sequence listing file attached to the present specification is provided below.

SEQ ID NO: 11

Sequence length: 9

Sequence type: DNA

Organism qualifier: Synthetic construct

Sequence name: Nucleotide sequence for CDR2 of anti-tau antibody (VL)

Sequence:
ctggtgtcc

SEQ ID NO: 12

Sequence length: 3
Sequence type: AA
Organism qualifier: Synthetic construct
Sequence name: Amino acid sequence for CDR2 of anti-tau antibody (VL)
Sequence:
LVS

**Claims**

1. A fusion comprising (i) an anti-tau antibody or an antigen-binding fragment thereof that specifically binds to tau protein and (ii) a blood-brain barrier receptor-binding peptide.

2. The fusion of claim 1, wherein the anti-tau antibody is directly coupled to the blood-brain barrier receptor-binding peptide without a linker.

3. The fusion of claim 1 or 2, wherein the blood-brain barrier receptor-binding peptide is selected from the group consisting of Angiopep-2 (APEP), RVG29, and TfR binding peptide (TfR).

4. The fusion of claim 3, wherein the APEP comprises the amino acid sequence of SEQ ID NO: 20, RVG29 comprises the amino acid sequence of SEQ ID NO: 22, and TfR comprises the amino acid sequence of SEQ ID NO: 24.

5. The fusion of claim 1 or 2, wherein the anti-tau antibody or antigen-binding fragment thereof comprises a heavy chain variable region (VH) that comprises heavy chain CDR1 comprising the amino acid sequence of SEQ ID NO: 2, heavy chain CDR2 comprising the amino acid sequence of SEQ ID NO: 4, and heavy chain CDR3 comprising the amino acid sequence of SEQ ID NO: 6; and a light chain variable region (VL) that comprises light chain CDR1 comprising the amino acid sequence of SEQ ID NO: 10, light chain CDR2 comprising the amino acid sequence of SEQ ID NO: 12, and light chain CDR3 comprising the amino acid sequence of SEQ ID NO: 14.

6. The fusion of claim 1 or 2, wherein the anti-tau antibody or antigen-binding fragment thereof binds to an epitope comprising the amino acids at positions 275 to 286 of the wild-type tau protein of SEQ ID NO: 25, in which the amino acid at position 280 is acetylated.

7. The fusion of claim 1 or 2, wherein the anti-tau antibody or antigen-binding fragment thereof comprises a heavy chain variable region comprising the amino acid sequence of SEQ ID NO: 8 and a light chain variable region comprising the amino acid sequence of SEQ ID NO: 16.

8. The fusion of claim 1 or 2, wherein the anti-tau antibody or antigen-binding fragment thereof comprises (i) a heavy chain variable region comprising an amino acid sequence selected from the group consisting of SEQ ID NO: 8, SEQ ID NO: 27, SEQ ID NO: 29, SEQ ID NO: 31, and SEQ ID NO: 33; and (ii) a light chain variable region comprising an amino acid sequence selected from the group consisting of SEQ ID NO: 16, SEQ ID NO: 35, SEQ ID NO: 37, and SEQ ID NO: 39.

9. The fusion of claim 1 or 2, wherein the anti-tau antibody comprises a light chain and a heavy chain, and the blood-brain barrier receptor-binding peptide binds to Fc region of the heavy chain.

10. The fusion of claim 1 or 2, wherein the fusion crosses a blood-brain barrier and specifically binds to tau protein.

11. The fusion of claim 1 or 2, wherein the anti-tau antibody or antigen-binding fragment thereof is selected from the group consisting of full-length antibody, Fab, scFv, F(ab')2, and Fv.

12. The fusion of claim 1 or 2, wherein the anti-tau antibody is an IgG antibody.

13. A polynucleotide encoding the fusion of claim 1 or 2.

14. An expression vector comprising the polynucleotide of claim 13.

15. A host cell comprising the expression vector of claim 14.

16. A method for producing the fusion of claim 1 or 2, comprising culturing a host cell that comprises a polynucleotide encoding the fusion of claim 1 or 2 or light chain and heavy chain polypeptides included in the fusion of claim 1 or 2.

17. A pharmaceutical composition for preventing or treating a neurodegenerative disease, comprising the fusion of claim 1 or 2.

18. The pharmaceutical composition of claim 17, wherein the neurodegenerative disease is a tau protein-mediated neurological disease.

19. The pharmaceutical composition of claim 17, wherein the neurodegenerative disease is selected from the group consisting of tauopathy, primary age-related tauopathy, chronic traumatic encephalopathy, progressive supranuclear palsy, corticobasal degeneration, frontotemporal dementia, Lytico-Bodig disease, Parkinson's disease, subacute sclerosing meningitis, lead encephalopathy, tuberous sclerosis, ganglioglioma, gangliocytoma, meningioangiomatosis, subacute sclerosing panencephalitis, Hallervorden-Spatz disease, and lipofuscinosis.

20. The pharmaceutical composition of claim 19, wherein the tauopathy is selected from the group consisting of Alzheimer's disease, progressive supranuclear palsy, corticobasal degeneration, Pick's disease, a group of related disorders collectively termed frontotemporal dementia with parkinsonism linked to chromosome 17 (FTDP-17), amyotrophic lateral sclerosis, Creutzfeldt-Jakob disease, dementia pugilistica, Gerstmann-Straussler-Scheinker syndrome, Lewy body disease, chronic traumatic encephalopathy, and Huntington's disease

21. The pharmaceutical composition of claim 17, wherein the pharmaceutical composition is administered via any one route of administration selected from the group consisting of intramuscular, intravenous, intraarterial, intraperitoneal, transdermal, subcutaneous, intradural, intracerebral, intracerebroventricular, intrapulmonary, or intranasal administration.

22. A method for preventing or treating a neurodegenerative disease, comprising administering an effective amount of the fusion of claim 1 or 2 to a subject in need of prevention or treatment of a neurodegenerative disease.

[FIG. 1A]

[FIG. 1B]

[FIG. 2]

BamH I (929)
Xho I (985)
BPPs
Xba I (1081)

BPPs_pcDNA3.1 (+)
5521 bp

[FIG. 3A]

BamH I (929)

Heavy chain_pcDNA3.1 (+)
6917 bp

Linker (4)
Xho I (2321)
BPP
Xba I (2477)

[FIG. 3B]

HindⅢ (911)

Light chain_pcDNA3.1 (+)
6071 bp

Xba I (1631)

[FIG. 4]

| iPSC expansion | NC/EC differentiation | BMEC | |
|---|---|---|---|
| mTeSR1 | UM | EC(w/RA) | EC |

D-3      D0         D6   D8   D9   D10

Seed hPSCs   Count hPSCs      Seed BMECs    TEER
(with Y27)   ($3\times10^5$ cells/well)    on transwell     ICC
                                                Drug test

[FIG. 5]

[FIG. 6]

Comparison of TEER values

[FIG. 7A]

Linker-free fusion (batch# 1, 2, 3)

[FIG. 7B]

Linker-free fusion (batch# 1, 2, 3)

[FIG. 8]

Linker-containing fusion (batch# 1 - 4)

## INTERNATIONAL SEARCH REPORT

| International application No. |
| --- |
| **PCT/KR2023/019923** |

| A. | CLASSIFICATION OF SUBJECT MATTER |
| --- | --- |

**C07K 16/18**(2006.01)i; **A61P 25/28**(2006.01)i; **A61K 39/00**(2006.01)i

According to International Patent Classification (IPC) or to both national classification and IPC

| B. | FIELDS SEARCHED |
| --- | --- |

Minimum documentation searched (classification system followed by classification symbols)

C07K 16/18(2006.01); A61K 39/00(2006.01); A61K 47/60(2017.01); A61K 47/64(2017.01); A61P 25/28(2006.01); C07K 16/28(2006.01); C07K 16/32(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 혈액-뇌 장벽(blood-brain barrier, BBB), 항-타우 항체(anti-Tau antibody), Angiopepe-2(APEP2), RVG29, TfR, 타우병증(tauopathy)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | KR 10-2019-0114907 A (HANMI PHARM. CO., LTD.) 10 October 2019 (2019-10-10) See abstract; paragraphs [0008], [0368]-[0369], [0373], [0377] and [0580]; example 5; and claims 1-3, 5 and 56-59. | 1-4,13-16 |
| Y | | 5-12,17-21 |
| DY | KR 10-2196840 B1 (ADEL, INC.) 30 December 2020 (2020-12-30) See abstract; paragraphs [0021], [0045] and [0176]; and claims 1, 3-4, 9, 11 and 14-15. | 5-12,17-21 |
| X | US 2019-0225699 A1 (BIOARCTIC AB) 25 July 2019 (2019-07-25) See abstract; and claims 1-30. | 1-3,13-16 |
| A | PARRASIA, S. et al. Peptides as Pharmacological Carriers to the Brain: Promises, Shortcomings and Challenges. Molecular Pharmaceutics. 29 September 2022, vol. 19, pp. 3700-3729. See abstract; tables 1-4; and page 3714. | 1-21 |

☑ Further documents are listed in the continuation of Box C.   ☑ See patent family annex.

| | | |
| --- | --- | --- |
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
| --- | --- |
| **12 March 2024** | **13 March 2024** |

| Name and mailing address of the ISA/KR | Authorized officer |
| --- | --- |
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/019923**

| C. | DOCUMENTS CONSIDERED TO BE RELEVANT | |
|---|---|---|
| Category* | Citation of document, with indication, where appropriate, of the relevant passages | Relevant to claim No. |
| A | KR 10-2021-0074279 A (DENALI THERAPEUTICS INC.) 21 June 2021 (2021-06-21)<br>See entire document. | 1-21 |

Form PCT/ISA/210 (second sheet) (July 2022)

**INTERNATIONAL SEARCH REPORT**

International application No.

**PCT/KR2023/019923**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

          ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
| --- |
| **PCT/KR2023/019923** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **22**
because they relate to subject matter not required to be searched by this Authority, namely:

Claim 22 pertains to a method for treatment of the human body, and thus pertains to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| | International application No. |
|---|---|
| | **PCT/KR2023/019923** |

| Patent document cited in search report | | | Publication date (day/month/year) | Patent family member(s) | | | Publication date (day/month/year) |
|---|---|---|---|---|---|---|---|
| KR | 10-2019-0114907 | A | 10 October 2019 | AR | 117563 | A1 | 18 August 2021 |
| | | | | EP | 3777894 | A1 | 17 February 2021 |
| | | | | TW | 201942138 | A | 01 November 2019 |
| | | | | TW | 201942139 | A | 01 November 2019 |
| | | | | US | 2021-0046189 | A1 | 18 February 2021 |
| | | | | WO | 2019-190291 | A1 | 03 October 2019 |
| | | | | WO | 2019-190293 | A1 | 03 October 2019 |
| KR | 10-2196840 | B1 | 30 December 2020 | AU | 2020-313751 | A1 | 10 February 2022 |
| | | | | BR | 112022000719 | A2 | 29 March 2022 |
| | | | | CA | 3143811 | A1 | 21 January 2021 |
| | | | | CL | 2022000087 | A1 | 10 March 2023 |
| | | | | CN | 114430744 | A | 03 May 2022 |
| | | | | EP | 4001305 | A1 | 25 May 2022 |
| | | | | GB | 2600599 | A | 04 May 2022 |
| | | | | IL | 289813 | A | 01 March 2022 |
| | | | | JP | 2022-541539 | A | 26 September 2022 |
| | | | | MX | 2022000603 | A | 20 May 2022 |
| | | | | TW | 202116801 | A | 01 May 2021 |
| | | | | US | 2022-0204601 | A1 | 30 June 2022 |
| | | | | WO | 2021-010712 | A1 | 21 January 2021 |
| US | 2019-0225699 | A1 | 25 July 2019 | AU | 2017-297804 | A1 | 24 January 2019 |
| | | | | BR | 112019000098 | A2 | 09 April 2019 |
| | | | | CA | 3028035 | A1 | 18 January 2018 |
| | | | | CN | 109476728 | A | 15 March 2019 |
| | | | | EP | 3484918 | A1 | 22 May 2019 |
| | | | | IL | 263773 | A | 28 February 2019 |
| | | | | JP | 2019-529345 | A | 17 October 2019 |
| | | | | JP | 2022-130646 | A | 06 September 2022 |
| | | | | KR | 10-2019-0039696 | A | 15 April 2019 |
| | | | | MA | 45684 | A | 22 May 2019 |
| | | | | MX | 2019000529 | A | 15 January 2020 |
| | | | | PH | 12018502451 | A1 | 30 September 2019 |
| | | | | RU | 2019102746 | A | 14 August 2020 |
| | | | | RU | 2019102746 | A3 | 30 November 2020 |
| | | | | SG | 10201912842 | A | 27 February 2020 |
| | | | | SG | 11201810801 | A | 30 January 2019 |
| | | | | US | 11498974 | B2 | 15 November 2022 |
| | | | | WO | 2018-011353 | A1 | 18 January 2018 |
| KR | 10-2021-0074279 | A | 21 June 2021 | AR | 115998 | A1 | 25 March 2021 |
| | | | | AU | 2019-326545 | A1 | 11 March 2021 |
| | | | | BR | 112021002953 | A2 | 11 May 2021 |
| | | | | CA | 3141815 | A1 | 27 February 2020 |
| | | | | CN | 113286610 | A | 20 August 2021 |
| | | | | EA | 202190603 | A1 | 14 July 2021 |
| | | | | EP | 3840781 | A1 | 30 June 2021 |
| | | | | IL | 280922 | A | 29 April 2021 |
| | | | | JP | 2021-534220 | A | 09 December 2021 |
| | | | | MX | 2021001976 | A | 08 June 2021 |
| | | | | SG | 11202101436 | A | 30 March 2021 |
| | | | | TW | 202017947 | A | 16 May 2020 |

Form PCT/ISA/210 (patent family annex) (July 2022)

**INTERNATIONAL SEARCH REPORT**
Information on patent family members

International application No.

**PCT/KR2023/019923**

| Patent document cited in search report | Publication date (day/month/year) | Patent family member(s) | | Publication date (day/month/year) |
|---|---|---|---|---|
| | | US 2022-0002436 A1 | | 06 January 2022 |
| | | WO 2020-041604 A1 | | 27 February 2020 |

Form PCT/ISA/210 (patent family annex) (July 2022)

## REFERENCES CITED IN THE DESCRIPTION

**Patent documents cited in the description**

- KR 20220171003
- KR 1020200086341
- KR 102196840

**Non-patent literature cited in the description**

- **FELGENHAUER**. *Klin. Wschr.*, 1974, vol. 52, 1158-1164
- Remington's Pharmaceutical Sciences and U.S. Pharmacopeia: National Formulary. Mack Publishing Company, 1984
- **LIPPMANN, E.** ; **AZARIN, S.** ; **KAY, J. et al.** Derivation of blood-brain barrier endothelial cells from human pluripotent stem cells. *Nat Biotechnol*, 2012, vol. 30, 783-791